# EUROPEAN PATENT APPLICATION

(11) **EP 2 722 005 A1**
(43) Date of publication of application: **23.04.2014**
(21) Application number: 13184778.2
(22) Date of filing: 17.09.2013
(51) Int. Cl.: A61B 7/04, A61B 5/021, A61B 5/024, A61B 5/00, H04R 1/28, H04R 19/01

(54) **Portable information terminal with microphone**

(30) Priority: 19.10.2012 JP 2012232317
(71) Applicant: FUJITSU LIMITED, Kawasaki-shi, Kanagawa 211-8588 (JP)
(72) Inventor: Watanabe, Kajiro, Koganei-shi, Tokyo 184-0013 (JP); Azami, Toshihiro, Kawasaki-shi, Kanagawa 211-8588 (JP)
(74) Representative: Ward, James Norman

(57) **Abstract**

A mobile information terminal including a directional microphone provided with a front port, a diaphragm and an electrode, wherein the microphone includes a front air chamber formed between a flexible thin film which covers an opening of the front port and the diaphragm, and a rear air chamber formed between a case and the electrode by hermetically covering the electrode with the case.
The mobile information terminal is equipped with two pulse wave detection sensors and a signal processing unit which computes the blood pressure based on the time difference of the pulse wave signals.

## Description

### FIELD

The present disclosure relates to a mobile information terminal that can detect a biometric signal.

### BACKGROUND

As the society is becoming more aging in developed countries, elderly people tend to pay more attention to their health. As a result, blood pressure meters capable of monitoring the blood pressure and the heart rate are widely distributed and mobile information terminals are also equipped with a pedometer so as to monitor health conditions. As an example, a device has been known in which a sensor unit provided with a low frequency condenser microphone and attached to a hollow frame is laid under the beddings of an experimental subject in sleeping so that the heart rate and the breathing rate, a rolling over time during sleep, and the frequency of snoring of the subject may be detected. See, for example, Japanese Laid-Open Patent Publication No. H11-28195.

Further, Japanese Laid-Open Patent Publication No. 2010-207553 discloses a mobile phone terminal equipped with a built-in microphone for communication capable of detecting a low frequency vibration and a health condition monitoring system which uses the mobile phone terminal as a pressure vibration detection unit detecting the pressure vibrations in a low frequency region such as a biometrical signal. In the mobile phone terminal and the health condition monitoring system disclosed in Japanese Laid-Open Patent Publication No. 2010-207553, in the microphone having a screen, and an electrode and a partitioning wall having a through-hole disposed in this order from the opening side within a housing, an air chamber is formed at the rear side of the partitioning wall so that the pressure variations in the low frequency region can be detected. In general, although a built-in microphone in the mobile phone terminal is able to detect only voice sound having a frequency range of 20 Hz or more, a rear chamber formed with a partitioning wall having the through-hole between a counter-electrode and a sealed end of a casing is provided in Japanese Laid-Open Patent Publication No. 2010-207553. With the rear chamber, the terminal and the system disclosed in Japanese Laid-Open Patent Publication No. 2010-207553 are able to detect the frequency up to a region of about 0.1 Hz in addition to voice signal. Moreover, the pressure change in indoor or inside the vehicle and biometric signals such as, for example, heart rate, breathing rate, rolling over time during sleep and the frequency of snoring may be detected as well.

### SUMMARY

A purpose of the embodements is for providing the mobile phone terminal with a built-in microphone for detecting a biometrical signal from any portion in the human body.

According to one aspect of the present disclosure, there is provided a mobile information terminal including a directional microphone provided with a front port, a diaphragm and an electrode. The microphone includes a front air chamber formed between a flexible thin film which covers the opening of the front port and the diaphragm, and a rear air chamber formed between a case and the electrode by hermetically covering the rear side of the electrode with the case.

### BRIEF DESCRIPTION OF DRAWINGS

FIG. 1A is a perspective view illustrating an example of a mobile information terminal to which the present disclosure is applied, FIG. 1B is a perspective view illustrating the structure of a condenser microphone provided in a mouthpiece of the mobile information terminal illustrated in FIG. 1A, and FIG. 1C is a cross-sectional view illustrating the structure of the condenser microphone illustrated in FIG. 1B.

FIG. 2A is a perspective view illustrating an exemplary structure of the condenser microphone provided with a front air chamber and a rear air chamber, and provided in the mobile information terminal of an embodiment, FIG. 2B is a longitudinal cross-sectional view of the microphone illustrated in FIG. 2A, and FIG. 2C is a cross-sectional view of the mobile information terminal illustrating the structure of the microphone of an embodiment of the present disclosure provided in the mouthpiece of the mobile information terminal.

FIG. 3A is a perspective view illustrating a mobile information terminal attached with a microphone according to a specific embodiment of the present disclosure, and FIG. 3B is a partial cross-sectional view illustrating a part of the microphone of the mobile information terminal illustrated in FIG. 3A.

FIG. 4A is a perspective view illustrating a mobile information terminal attached with a microphone according to another embodiment of the present disclosure, and FIG. 4B is a longitudinal cross-sectional view illustrating the mobile information terminal of FIG. 4A.

FIG. 5A is a perspective view illustrating the mobile information terminal according to an embodiment where a pulse wave detection sensor is provided at a location at which a thumb is placed when the mobile information terminal of FIG. 3A is held by a left hand, FIG. 5B is a front view illustrating the configuration of the pulse wave detection sensor as an embodiment, and FIG. 5C is an explanatory view illustrating the operation of the pulse wave detection sensor of FIG. 5B.

FIG. 6A is a view illustrating a usage example when the mobile information terminal of FIG. 5A is placed on the chest to measure the blood pressure, and FIG. 6B is a view illustrating a usage example when the mobile information terminal of FIG. 5A is placed on the neck to measure the blood pressure.

FIG. 7 is a diagram illustrating the configuration of a circuit measuring the blood pressure in the mobile information terminal of FIG. 5A.

FIG. 8 is a flowchart illustrating the operation for calculating the blood pressure by the signal processing unit of FIG. 7.

FIG. 9 is a perspective view of the mobile information terminal illustrating an embodiment in which a pulse wave detection sensor provided at a location where the thumb is placed on the mobile information terminal of FIG. 5A is omitted.

FIG. 10A is a view illustrating an example where the mobile information terminal of FIG. 9 is placed on the chest to measure the blood pressure, and FIG. 10B is a view illustrating an example where the mobile information terminal of FIG. 9 is placed on the neck to measure blood pressure.

FIG. 11 is a block diagram illustrating the configuration of a circuit that measures the blood pressure in the mobile information terminal of FIG. 9.

FIG. 12 is a flowchart illustrating the operation of the signal processing unit of FIG. 11 for calculating the blood pressure.

### DESCRIPTION OF EMBODIMENTS

In the mobile phone terminal and the health condition monitoring system disclosed in the Japanese Laid-Open Patent Publication No. 2010-207553, since an opening is formed at the front side of a screen provided within the housing, there were problems that air which drives the screen is easy to leak, the screen may not be driven sufficiently, and the sensitivity in the low frequency region may not be sufficiently obtained.

According to an aspect of the present disclosure, there is provided a mobile information terminal capable of measuring a pulse wave even with an infinitesimal pressure variation, using a microphone for communication built-in in the mobile information terminal without being limited to the part of a human body on which the microphone is to be placed. According to another aspect of the present disclosure, there is provided a mobile information terminal in which a sensor capable of detecting the pressure variation in the low frequency region of two sites is installed to detect the blood pressure.

According to yet another aspect of the present disclosure, there is provided a mobile information terminal equipped with a built-in microphone including a front port, a diaphragm and an electrode. In the microphone, a front surface side of the front port is covered with a flexible thin film to form a front air chamber between the front port and the diaphragm and also, a rear of the electrode is covered with a casing to be hermetically sealed to form a rear air chamber between the electrode and the casing, such that the pressure variations in a low frequency region having a frequency of a voice band or less can be detected.

According to yet another aspect of the present disclosure, there is provided a mobile information terminal equipped with a built-in microphone including a front port, a diaphragm and an electrode. In the microphone, the front side of the front port is covered with a flexible thin film to form a front air chamber between the front port and the diaphragm, and at the same time, the rear side of the electrode is covered with a casing to be hermetically sealed to form a rear air chamber between the electrode and the casing, such that the pressure variation may be detected in a low frequency region having a frequency of a voice band or less. The microphone may be provided in the mouthpiece of the mobile information terminal as the first sensor, and at the same time, a sensor capable of detecting the pulse wave of the finger is provided as the second sensor in a position of the mobile information terminal where the user is supposed to hold the housing with the fingers. The mobile information terminal is also provided with a signal processing unit configured to detect the time difference of the biometric signals detected by the first and second sensors at the same time range.

Hereinbelow, embodiments of the present disclosure will be described in detail based on specific embodiments with reference to the accompanying drawings.

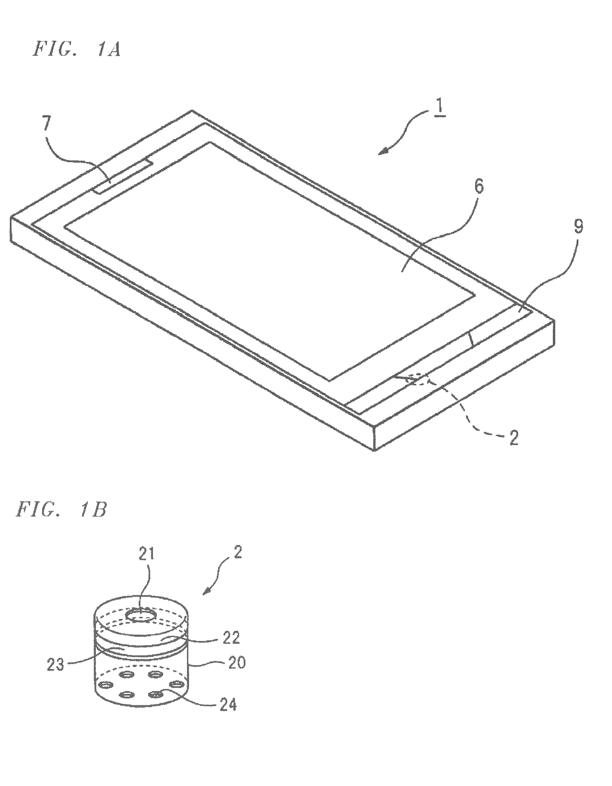

FIG. 1A illustrates an example of a mobile information terminal 1 to which the present application is applied. In the mobile information terminal 1, a display 6 is disposed at the central part thereof, a microphone 2 is disposed below the display 6 for sound transmission, and a speaker 7 is disposed above the display 6 for sound producing. The reference numeral 9 denotes a functional button.

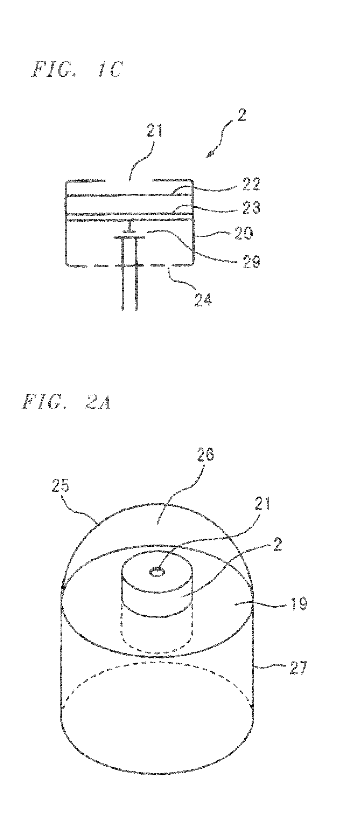

FIG. 1B is a perspective view illustrating the configuration of the microphone 2 provided in a mouthpiece of the mobile information terminal 1 as illustrated in FIG. 1A, and FIG. 1C illustrates the cross-section of the microphone 2 as illustrated in FIG. 1B. The microphone 2 is generally a directional electret condenser microphone, and the outside air vibration (sound wave) through a front port 21 disposed at the front end of a casing 20 causes a diaphragm 22 to vibrate such that a gap between the diaphragm 22 and an electrode 23 varies. When the diaphragm 22 is vibrating, the capacitance between the electrode 23 and the diaphragm 22 varies and the capacitance variation is converted into an electrical signal. As a result, the sound wave is converted into an electrical signal and the electrical signal in turn is amplified by an amplifier 29 such as an FET to be extracted outside. A back port 24 is provided at the rear end portion of the casing 20 of the microphone 2.

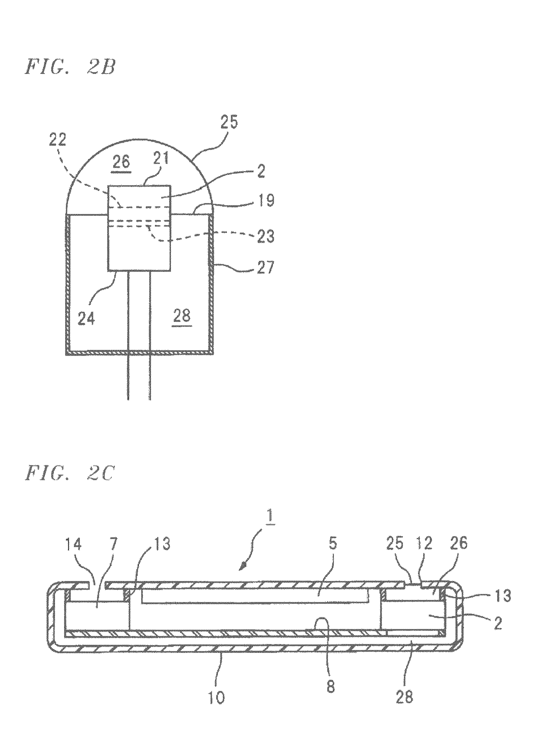

In the microphone 2 having the configuration as described above, as illustrated in FIGS. 2A and 2B, the front and rear of the microphone 2 is partitioned with a partitioning wall 19 and, the front side of the partitioning wall 19 is covered with a flexible thin film (hereinafter, referred to as a flexible film) 25 to form a front air chamber 26 between the flexible film port and the diaphragm. Further, the rear side of the partitioning wall 19 is covered with a case 27 to be hermetically sealed to form a rear air chamber 28 between the electrode and the case. The partitioning wall 19 is not flexible. The variation of outside air is transmitted to the diaphragm through the front air chamber 26 and the front port 21. The rear air chamber 28 becomes a space separated from the front air chamber 26 by the partitioning wall 19 and the case 27.

In the microphone 2, the outside air vibration (sound wave) reaches the diaphragm 22 along with each of the front port 21 and the back port 24. When the frequency of sound is low as in the ultra low frequency sound less than the audible frequency band, the atmospheric pressure variation is slow and thus, the atmospheric pressure variation from the front port 21 and the atmospheric pressure variation from the back port 24 are generated almost in phase to pressurize the diaphragm 22 each other from the frontward and rearward, causing the vibration of the diaphragm 22 to become smaller. As a result, the sensitivity of the microphone 2 is reduced. In the present disclosure, the rear air chamber 28 is enclosed to be sealed and separated from the front air chamber26 and thus, the atmospheric pressure variation through the front port 21 becomes independent from the atmospheric pressure variation through the back port 24. Therefore, even for ultra low frequency sound, a phenomenon that the diaphragm 22 is pressurized each other from the frontward and rearward in phase does not happen and a phenomenon that the sensitivity of the microphone 2 is reduced does not happen. Further, due to the presence of the front air chamber 26, the flow rate of the air to be supplied to the front port 21 may be increased and the vibration of the diaphragm 22 in the ultra low sound range may be increased. Therefore, the pressure variation may be detected in a low frequency region which is lower than a voice band.

FIG. 2C illustrates an embodiment in which the microphone 2 having a structure illustrated in FIGS. 2A and 2B is built in the mobile information terminal 1. The microphone 2 provided in a mouthpiece of the mobile information terminal 1 is used as a microphone which detects the pressure variation of sound in a low frequency region. In the present embodiment, the front side of the microphone 2 is attached to the housing 10 through a separator 13. The separator 13 is configured to prevent air from passing between the housing 10 and the microphone 2 using, for example, a packing made of rubber or urethane. Also, in the present embodiment, a flexible film 25a is provided at a microphone hole 12 of the housing 10. The flexible film 25 is a thin stretchable film such as a plastic film, and may be made of materials, such as, for example, vinyl chloride, acrylic or polycarbonate.

A space surrounded by the flexible film 25, the housing 10, the separator 13, and a front surface of the microphone 2 corresponds to the front air chamber 26. The rear air chamber 28 is formed at the rear surface side of the microphone 2 using a space within the housing 10. When the mobile information terminal 1 is a smart phone, the speaker 7 is also attached into the housing 10 and the air hole 14 is also disposed at the front surface of the housing 10. Accordingly, in order to hermetically seal the rear air chamber 28, the speaker 7 is attached to a circuit board 8 within the housing 10 through the separator 13. According to the present embodiment, even the smart phone terminal or the mobile phone terminal in which the air hole 14 is also disposed on the part of the speaker 7 may be able to detect the ultra low frequency sound as well. Reference numeral 5 denotes a display.

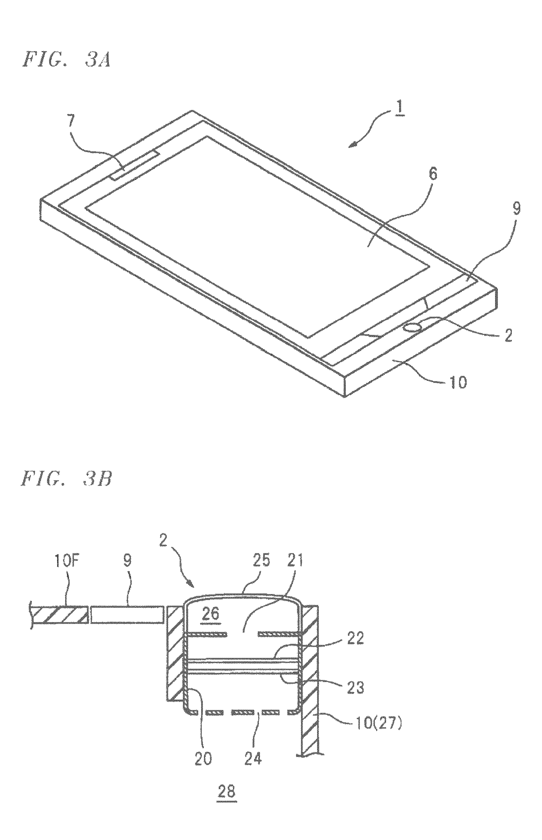

FIG. 3A illustrates a perspective view of the mobile information terminal 1 attached with the microphone 2 according to a specific embodiment of the present disclosure, and FIG. 3B illustrates a partial cross-sectional view of a part at which the microphone 2 of the mobile information terminal 1 illustrated in FIG. 3A is installed. In the present embodiment, the microphone 2 is installed on a part of the housing 10 which corresponds to a further end portion side than the functional buttons 9 of the mobile information terminal 1. The microphone 2 having the structure illustrated in FIG. 1C is used as the microphone 2, and the casing 20 of the microphone 2 is embedded into the housing 10. Accordingly, the flexible film 25 attached to the upper side of the front port 21 is exposed to the upper surface 10F of the housing 10. The surface of a flexible film 25 is bulged out from the upper surface 10F of the housing 10 and is adapted to be easily adhered to a human body. A space between the flexible film 25 and the front port 21 corresponds to the front air chamber 26. The case 27 which forms the rear air chamber 28 of the microphone 2 is the housing 10 of the mobile information terminal 1 in the present embodiment. Further, although not illustrated, a gap between the functional button 9 of the mobile information terminal 1 and the upper surface 10F of the housing 10 is adapted to be hermetically maintained.

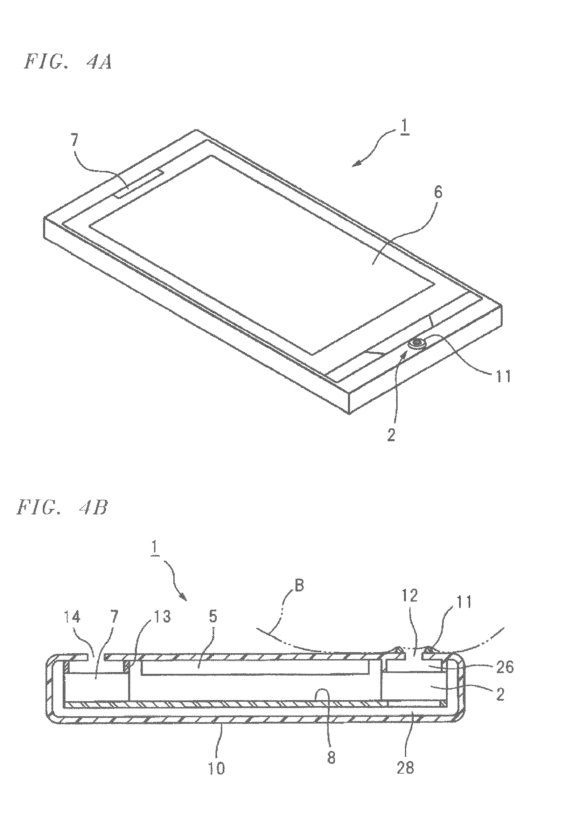

FIG. 4A is a perspective view illustrating the mobile information terminal 1 attached with the microphone 2 according to another embodiment of the present disclosure, and FIG. 4B is a longitudinal cross-sectional view of the mobile information terminal illustrated in FIG. 4A. In the present embodiment, a flexible film is not attached to the front air chamber 26, and an annular projection part 11 is disposed around the microphone hole 12. The annular projection part 11 is formed of a flexible material such as rubber or urethane. In the present embodiment, the annular projection part 11 is allowed to directly contact with a target object for detection such as the skin of a human body B illustrated with the two-dotted chain line, such that an air chamber is configured by the annular projection part 11 and the subject, resulting in the front air chamber 26. Other structure is the same as that of the mobile information terminal 1 of the embodiment illustrated in FIG. 2C and thus, the same reference numerals are given to the same element members and the descriptions thereof will be omitted.

According to the embodiments illustrated in FIGS. 4A and 4B, since the front air chamber 26 is hermetically sealed against outside air due to the contact of the annular projection part 11 and the target object, the rear air chamber 28 may not necessarily be hermetically sealed. That is, when the annular projection part 11 is disposed around the air hole 14 disposed at the front side of the annular projection part 11, and the annular projection part 11 around the air hole 14 is allowed to directly contact with a target object for detection such as the skin of the human body B at the time of measuring, it would not cause any problem even if the separator 13 is not provided in the speaker 7. According to the present embodiment, in terms of detecting the vibration waveforms, such as the heartbeat or the breathing in a state where the object which is vibrating can be directly touched, the detection of vibration waveforms may be implemented with a configuration simpler than the embodiment 1 described above.

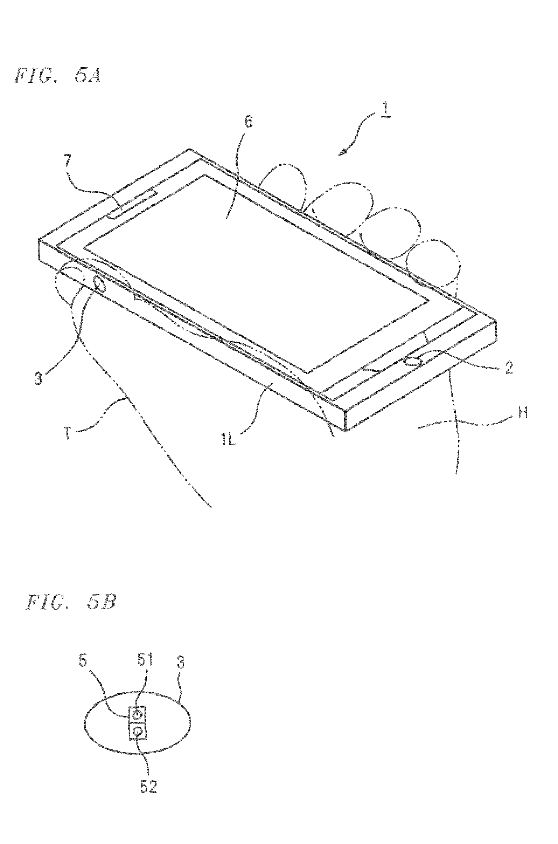

FIG. 5A is a perspective view of the mobile information terminal illustrating an embodiment where a pulse wave detection sensor 3 is provided on a position on which the fingertip of a thumb T is placed when the mobile information terminal 1 illustrated in FIG. 3A is held by the left hand H of a user. When the mobile information terminal 1 is held by the left hand H of a user, the position on which the thumb T is placed corresponds to a left side surface 1L of the housing 10 and thus, the pulse wave detection sensor 3 is installed at the left side surface 1L. The microphone 2 having the same configuration as the microphone 2 described with respect to FIG. 3B may be used as the pulse wave detection sensor 3, but an infrared sensor 5 using infrared ray as illustrated in FIG. 5B may be used as well, instead of the microphone 2. The infrared sensor 5 includes a light emitting element 51 provided with an LED which corresponds to a light source and a light receiving element 52. The pulse wave detection sensor 3 may be provided at a right side surface.

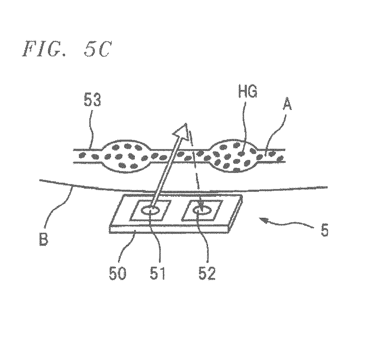

FIG. 5C is an explanatory view illustrating operations of an infrared sensor 5e which is a pulse wave detection sensor which is an infrared sensor 5e illustrated in FIG. 5B. The light emitting element 51 and the light receiving element 52 are provided on a circuit board 50. Infrared ray light is irradiated from the light emitting element 51 toward the human body B and the reflected light from the human body B is received by the light receiving element 52. The flow rate of blood "A" flowing through the artery 53 changes with the heartbeat from the heart, and a part of the artery 53 where a large amount of blood flows is dilated. Hemoglobin HG is present in blood "A" flowing through the artery 53, and has a characteristic to absorb the light that was introduced from the light emitting element 51. Therefore, it is possible to detect the quantity of the reflected light by the light receiving element 52 in a time sequence to acquire a pulse wave flow.

Here, the wavelength appropriate for identifying the degree of light absorption in hemoglobin corresponds to the near infrared wavelength region of 700 nm to 900 nm. In the near infrared wavelength region, the light absorption by water or hemoglobin or other living organism compound is small and thus light easily passes through the living organism. In the meantime, the wavelength used in the IrDA infrared communication device provided in the mobile phone is similarly near infrared wavelength region of 850 nm to 900 nm. However, the light emitting element and light receiving element according to IrDA method is formed in an module structure optimized for IrDA infrared communication device, but is not formed in a structure where the light is emitted to a living organism and reflected from blood in blood vessels to be received. Therefore, the light emitting element and light receiving element according to an IrDA method may not detect the pulse wave. Accordingly, an IrDA infrared sensor may not be used as a sensor to see a pulse wave. The infrared sensor 5 which is a pulse wave detection sensor is separately provided from an infrared sensor used for an IrDA infrared communication device.

The mobile information terminal 1 equipped with two pulse wave detection sensors 2, 3 as illustrated in FIG. 5A is used such that the pulse wave of a part of a human body on which the pulse wave detection sensor 2 of the mobile information terminal 1 is placed and a pulse wave of the thumb T, that is, two pulse waves of the parts of human body spaced apart from each other, may be detected. Also, when the parts of human body spaced apart from each other can be measured, the blood pressure may be computed and obtained by obtaining the time difference in detection between the detected pulse waves.

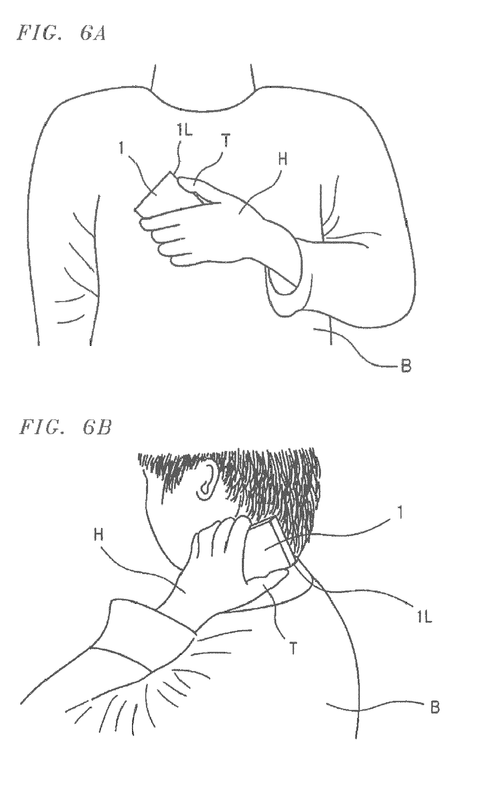

FIG. 6A is a view illustrating an example when the mobile information terminal 1 illustrated in FIG. 5A is held by the left hand H of a user and the mouthpiece is placed on the chest to measure the blood pressure, and FIG. 6B is a view illustrating an example when the mobile information terminal 1 illustrated in FIG. 5A is held by the left hand H of a user and the mouthpiece is placed on the neck to measure the blood pressure. In these examples, the thumb T is needed to be placed on the pulse wave detection sensor 3 disposed on the left side surface 1L of the mobile information terminal 1, and the mouthpiece is needed to be placed on a position where the pulse wave of the human body B can be detected.

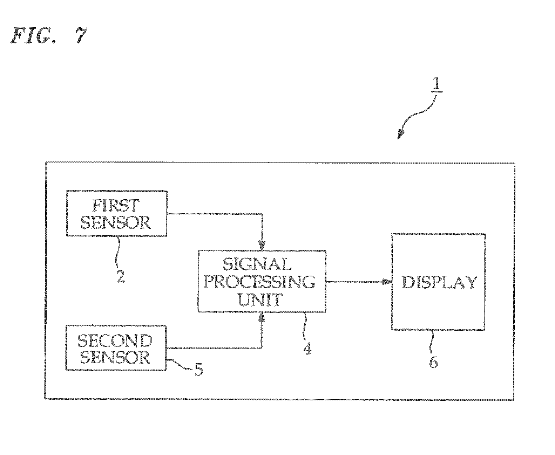

FIG. 7 is a diagram illustrating the configuration of a circuit measuring the blood pressure in the mobile information terminal 1 illustrated in FIG. 5A. The outputs of the first sensor (microphone) 2 and the second sensor (infrared sensor) 5 are input to the signal processing unit 4. The signal processing unit 4 computes the blood pressure based on the time difference in detection between pulse wave signals input from two sensors 2, 3 and displays a blood pressure level obtained by computation on the display 6. The second sensor may be the microphone 2 as well.

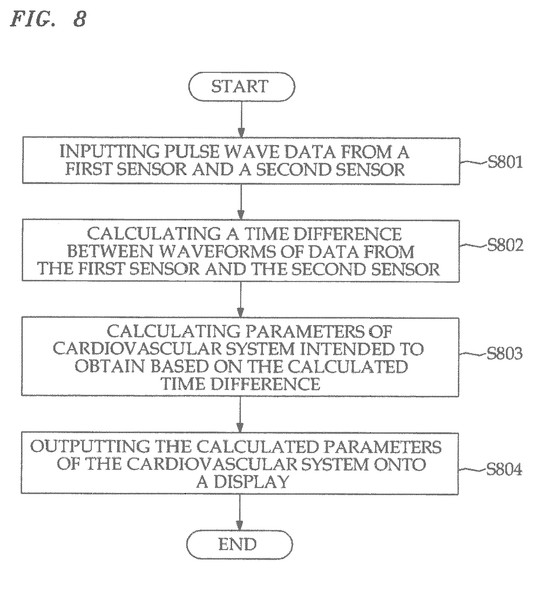

FIG. 8 is a flowchart illustrating the operation of the signal processing unit 4 illustrated in FIG. 7 for calculating the blood pressure. When measuring starts, pulse wave data is input from the first sensor and the second sensor at step 801. Data input may be made regardless of whether data is input in real time or by reading the buffered data. At step 802, the signal processing unit 4 calculates the time difference between the waveforms of data from the first sensor and the second sensor. The time difference between the waveforms of data may be calculated by comparing the feature points of a waveform, for example, peaks or zero crossing points between two data. Further, the time difference may be obtained based on a timing at which the value of signal is reduced from the peak of the waveform of data by a certain ratio. For example, the time difference may be calculated based on a time point which corresponds to 50% of amplitude from an upper peak to a lower peak.

Also, at step 803, the signal processing unit 4 calculates the parameter of the cardiovascular system obtained based on the calculated time difference. For example, when the first sensor (microphone of the mouthpiece) detects a pulse wave at a position in the vicinity of the heart and the second sensor detects a pulse wave in the fingertip of the thumb, the time it takes for blood pumped out of the heart to reach the fingertip of the thumb may be determined. Therefore, the parameters correlated to the blood pressure may be calculated. Finally, the signal processing unit 4 outputs the parameter of the cardiovascular system (blood pressure level) calculated at step 804 on a display provided on the mobile information terminal, and the process routine ends. Further, the calculated parameter of the cardiovascular system (blood pressure level) may be transmitted to other medical equipment via radio waves from the mobile information terminal.

Further, a method for obtaining the blood pressure from the change of pulse wave arrival time between two points of human body is described in the following reference literature 1.
(Reference literature 1) W. Chen, T. Kobayashi, S. Ichikawa, Y. Takeuchi, T. Togawa, "Continuous estimation of systolic blood pressure using the pulse arrival time and intermittent calibration," Medical & Biological Engineering & Computing vol.3, pp.569-574, 2000.

According to the reference literature 1, the blood pressure Pb of the reference state and the arrival time Tb is obtained in advance and then, the blood pressure Pe may be calculated using a change ratio ΔT of an arrival time to the arrival time Tb (see formula (10) of the of reference literature 1). According to the reference literature 1, the blood pressure Pe may be obtained in such a manner that the time difference calculated at step 802 of FIG. 8, that is, the arrival time is set to T, and the change of the arrival time ΔT is set as ΔT=T-Tb, and the formula (10) of the of reference literature 1 is used at step 803.

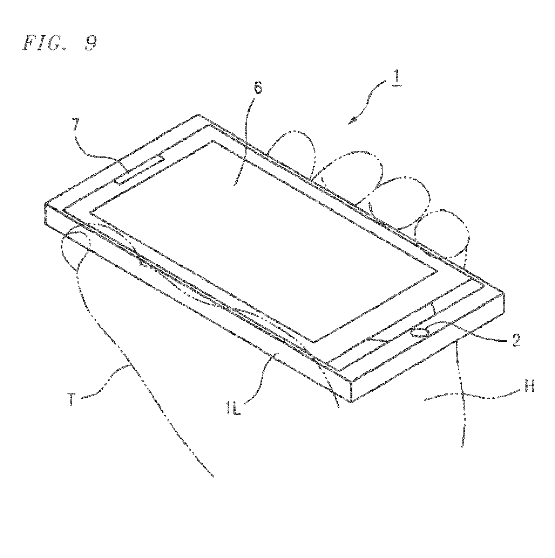

FIG. 9 is a perspective view of the mobile information terminal illustrating an embodiment where the blood pressure is obtained using only the pulse wave detection sensor 2 in the mobile information terminal 1 equipped with the pulse wave detection sensors 2, 3 illustrated in FIG. 5A and thus, the pulse wave detection sensor 3 is omitted. For example, when the waveform of the pulse wave of a position of a human body on which the pulse wave detection sensor 2 of the mobile information terminal 1 is placed is used in an algorithm of obtaining the blood pressure from the waveform of the pulse wave according to Japanese Laid-Open Patent Publication No. 2002-320593, the blood pressure may be obtained with only the pulse wave by the first sensor (microphone of the mouthpiece) and thus, the second sensor becomes unnecessary.

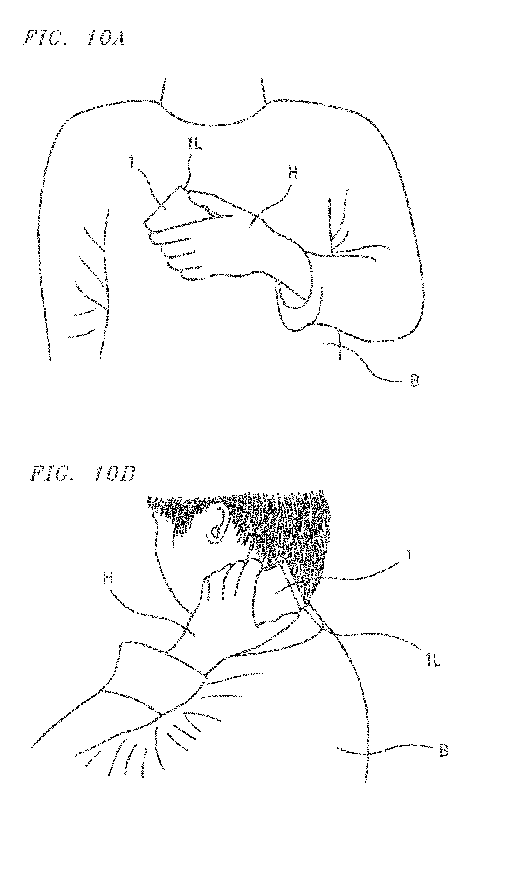

FIG. 10A is a view illustrating an example when the mobile information terminal 1 illustrated in FIG. 9 is held by the left hand H of a use and the mouthpiece is placed on the chest to measure the blood pressure, and FIG. 10B is a view illustrating an example when the mobile information terminal illustrated 1 in FIG. 9 is held by the left hand H of a user and the mouthpiece is placed on the neck to measure the blood pressure. In these examples, as opposed to the embodiments illustrated with respect to FIGS. 6A and 6B, the position of the thumb becomes free.

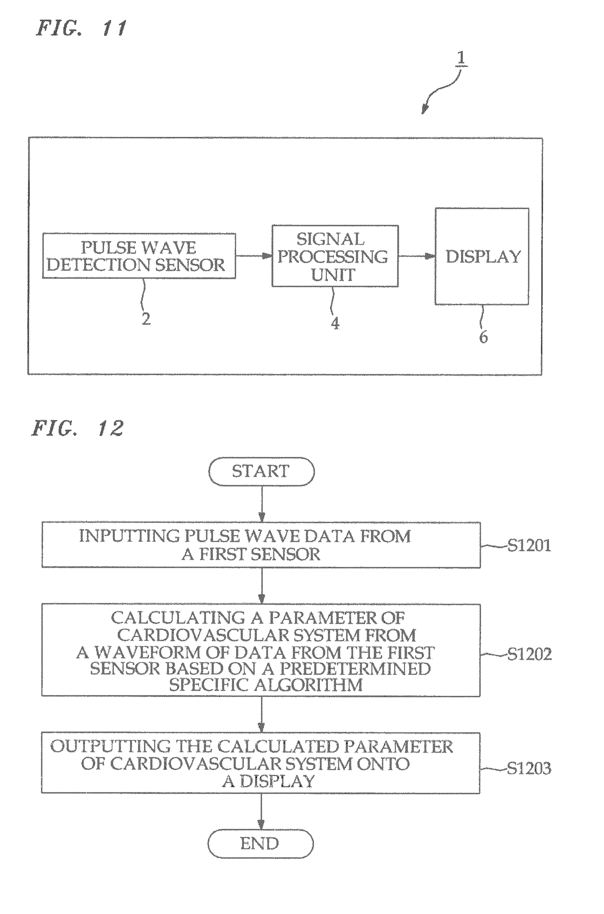

FIG. 11 is a diagram illustrating the configuration of a circuit measuring the blood pressure in the mobile information terminal 1 illustrated in FIG. 9. The output of the pulse wave detection sensor (first sensor; microphone) 2 is input to the signal processing unit 4. The signal processing unit 4 computes the blood pressure based on a signal waveform of pulse wave signal input from the pulse wave detection sensor 2 and displays the blood pressure level obtained by computation on the display 6.

FIG. 12 is a flowchart illustrating the operation of the signal processing unit 4 illustrated in FIG. 11 for calculating the blood pressure. When measuring starts, the pulse wave data is input from the first sensor (pulse wave detection sensor) at step 1201. Data input may be made regardless of whether data is input in real time or by reading the buffered data. At step 1102, the signal processing unit 4 calculates the parameter of the cardiovascular system from waveforms of data from the first sensor based on a specific algorithm determined in advance. Data from the first sensor is a pulse wave data, and the blood pressure may be calculated from the waveforms of the pulse wave data by the specific algorithm.

Finally, the signal processing unit 4 outputs the parameter of the cardiovascular system (blood pressure level) calculated on a display provided on the mobile information terminal at step 1203, and the process routine ends. Further, the calculated parameter of the cardiovascular system (blood pressure level) may be transmitted to other medical equipment via radio waves from the mobile information terminal.

Here, a method for obtaining the blood pressure from change of pulse wave arrival time at one point of the human body is described in the Japanese Laid-Open Patent Publication No. 2002-320593

According to an artery blood pressure measurement method described in the reference literature 2, it is possible to calculate the maximum blood pressure and the minimum blood pressure from artery waveforms and differential artery waveforms produced by differentiating the artery waveforms, as described hereinbelow.

The zero crossing point computational process is performed for obtaining one period of time T of the artery differential waveform in each heartbeat, the time Ta from a zero crossing point corresponding to the minimum of the artery waveform to a zero crossing point corresponding to the maximum of the artery waveform, and the time Tb produced by subtracting the time Ta from the time T, using the zero crossing point of the differential artery waveform for each heartbeat. In the blood pressure computational process, the number of pulses during one minute "n" is obtained using an equation n=(60/T) using values of the time T, time Ta and time Tb obtained by the zero crossing point computational process. The maximum blood pressure Ph for each heartbeat is calculated from a relative equation {Ph*n=-A*LOG(Ta*Tb)-B}, where A and B are integers and LOG is a notation for a common logarithm, and the minimum blood pressure Pl for each heartbeat is calculated from a relative equation {n*n/Pl=C*(Tb*Tb)D}, where C and DB are integers.

As described above, in the present disclosure, the flexible film which does not let air pass through is installed to cover the front air chamber of the microphone of the mobile information terminal and thus, the vibration of the fingers tightly contacted to the flexible film is converted into a pressure change of air inside the front air chamber by the flexible film. Also, the microphone detects the pressure change in air transferred from the front air chamber and it becomes possible to detect a pulse. Also, the microphones are installed on the front surface and one side surface of the mobile information terminal, such that the blood pressure may be detected by the mobile information terminal when one of the microphones is placed on and tightly contacted to the carotid arteries and the other of the microphones is placed on and tightly contacted to the fingers. Further, the pulse wave may be detected at the fingertip of the thumb even if the infrared sensor is installed instead of the microphone installed at the side surface of the housing of the mobile information terminal..

Further, when an algorithm is used for obtaining the blood pressure from the waveform of the pulse wave at one position of the human body, such a microphone is needed to be installed only one site of the mobile information terminal in order to detect the blood pressure.

Further, similarly to the mobile information terminal 1 illustrated in FIG. 5A, the mobile information terminal 1 in which the pulse wave detection sensor 3 is provided on the position on which the fingertip of the thumb T is placed is able to detect the blood pressure with the pulse wave detection sensor 3 by using the algorithm of obtaining the blood pressure from the waveform of the pulse wave at one position of the human body. That is, it is possible to detect the blood pressure by the pulse wave detection sensor 3 located at the position on which the fingertip of the thumb T is placed while communicating by holding the mobile information terminal 1 by the left hand H.

## Claims

1. A mobile information terminal comprising:
a directional microphone provided with a front port, a diaphragm and an electrode,
wherein the microphone includes a front air chamber formed between a flexible thin film which covers an opening of the front port and the diaphragm, and a rear air chamber formed between a case and the electrode by hermetically covering a rear of the electrode with the case.

2. The mobile information terminal according to claim 1, wherein the case is the mobile information terminal of claim 1, and the case adopts an hermetical structure against outside air.

3. The mobile information terminal according to claim 2, further comprising:
a sensor provided in the case; and
a signal processing unit configured to detect a time difference between biometric signals detected at the same time zone by the directional microphone and the sensor.

4. The mobile information terminal according to claim 3, wherein the signal processing unit calculates a blood pressure level of a user of the mobile information terminal based on the time difference between the detected biometric signals.
